Europäisches Patentamt

European Patent Office

Office européen des brevets

(19)

(11) Publication number: **0 147 780**
A2

(12)

# EUROPEAN PATENT APPLICATION

(21) Application number: **84115782.9**

(22) Date of filing: **19.12.84**

(51) Int. Cl.⁴: **A 61 K 9/32**
**A 61 K 9/52, A 61 K 9/02**

(30) Priority: **03.01.84 US 567835**

(43) Date of publication of application:
**10.07.85 Bulletin 85/28**

(84) Designated Contracting States:
**CH DE FR GB IT LI NL**

(71) Applicant: **MERCK & CO. INC.**
**126, East Lincoln Avenue P.O. Box 2000**
**Rahway New Jersey 07065(US)**

(72) Inventor: **Bondi, Joseph V.**
**3825 Kratz Road**
**Pennsylvania 19426(US)**

(74) Representative: **Abitz, Walter, Dr.-Ing. et al,**
**Abitz, Morf, Gritschneder, Freiherr von Wittgenstein**
**Postfach 86 01 09**
**D-8000 München 86(DE)**

(54) Drug delivery device.

(57) Polyvinyl alcohol for use as a film coating, a matrix for monolithic device or as a barrier coating for the preparation of controlled release drug delivery systems.

EP 0 147 780 A2

16923

TITLE OF THE INVENTION
DRUG DELIVERY DEVICE

BACKGROUND OF THE INVENTION

The invention relates to a novel method and compositions for release of drugs through a rate limiting barrier by coating a formulation with polyvinyl alcohol, which serves as a membrane for said drugs or acts as a barrier film which will selectively permit passage of the desired species.

DESCRIPTION OF THE PRIOR ART

The literature contains numerous references to polymer films acting as barrier coatings. Films on oral dosage forms may dissolve freely in g.i. fluids or dissolve selectively at specific pH conditions. Certain films function by permitting only water to permeate the product giving rise to an increased osmotic pressure. Other films may be solubilized by the action of enzymes or slow hydrolysis of polymer in contact with body fluids.

Cellulose acetate, a polymer known throughout the prior art acts as a reverse osmotic membrane which selectively permits water to pass but prevents passage of solutes. Its solubility requires that solutions be prepared in volatile organic solvents, thus leading to a hazardous and expensive process for preparing films, tablets or granules. See U.S. Patent Nos. 3,917,813 and 3,954,959 which teach oral drug preparations having a protracted release rate and comprising a pharmaceutically active component, one or more buffer acids or salts coated with ethyl cellulose and an acrylic polymer, respectively, in order to control the rate of diffusion and the release of the coated active substance. The references also teach use of conventional plasticizers which may give rise to interaction with the drug substance and further may impose shelf life limitations on the product.

SUMMARY OF THE INVENTION

The invention describes a delivery device which controls the release of drugs and rate limiting barriers for drug delivery systems.

Accordingly, it is an object of this invention to:

Provide a novel drug delivery device for dispensing of a composition of matter thereby producing a beneficial effect, said device alleviating the aforesaid disadvantages associated with the prior art's devices;

Provide a novel dispensing device for dispensing a composition of matter at a controlled rate for a prolonged period of time;

Provide a novel device which will permit high concentration of an active agent(s) contained therein, and said high concentration will not exhibit the tendency to be leached from the device nor have its potency decreased by chemical breakdown;

Provide a novel delivery device that contains a drug which can be used as an effective solute to exhibit an osomotic pressure gradient against external fluid;

Provide a novel delivery device for the administration of locally acting or systematically acting drugs to produce a physiologic or pharmacologic effect and release the drug at a rate that does not vary with time;

Provide a device for delivering an active agent having a variety of release rates ranging from very slow to very high by using polyvinyl alcohol as a wall forming material in combination with an active agent or mixture thereof; and

Other objects, features, and advantages of the invention will be apparent to those skilled in the art from the detailed description of the specification and the accompanying claims.

DETAILED DESCRIPTION OF THE INVENTION

The novel device of this invention is a delivery system shaped and sized for oral ingestion, rectal or vaginal insertion for delivery of a drug or pharmaceutically acceptable salts thereof or other beneficial substances comprising a nucleus (core) consisting of the active agent(s), optionally one or more buffers and a polyvinyl alcohol film for coating of tablets, suppositories and granules. Another novel device of this invention is a delivery system shaped and sized for oral ingestion, rectal or vaginal insertion for delivery of a drug or pharmaceutically acceptable salt thereof or other beneficial substances dispersed homogeneously throughout a matrix composed of polyvinyl alcohol. The compositions of this invention are prepared by conventional methods utilized in the prior art. However, unlike other coatings such as ethylcellulose which do not hydrate and acrylic polymers which may either remain insoluble or dissolve at different sites along the g.i. tract, polyvinyl alcohol as a coating remains intact and in addition thereto maintains an aqueous envelope around the dosage form or swells around the dosage form in an aqueous environment. This hydrated state permits controlled passage of drug to the external environment of the dosage form. In addition, this state as well prevents high drug concentration from coming in direct contact with the intestinal mucosa. Thus, polyvinyl alcohol remains an integral part of the dosage form and will not rupture due to hydration of the core tablet as is the case with ethylcellulose nor dissolve as with acrylate type polymers.

In its operation, the device is introduced to the appropriate site such as the rectum, gastrointestinal tract or vagina. The polyvinyl alcohol film membrane is permeable to water and therefore water passes through and causes the overall rate of drug delivery to be determined by the buffer which aids in ionization of said drug and alteration of the pH, thereby controlling the relative proportion of ionized to non-ionized drug substance.

0147780

The substance forming the selective film coating membrane is polyvinyl alcohol and its thickness is inversely proportional to the desired rate of release of the drug. As an example, a 25% increase in polyvinyl alcohol film thickness resulted in a 35% decrease in the transport of timolol through the membrane.

Polyvinyl alcohol "super hydrolyzed" grade exhibits solubility in hot (boiling) water but is insoluble in cold or warm water. Solutions can be prepared that remain as solutions at room temperature. The polyvinyl alcohol used in this invention, however, does imbibe water or fluids at temperatures below its solubility temperature to form a tough, transparent, gel like film containing up to 50 percent water.

Various active agents provide beneficial effects when administered to patients. Such agents which can be made more useful by controlled selective delivery through a protective polyvinyl alcohol membrane coating in accordance with the present invention, are exemplified by, but not limited to, the following classes of agents:

(a)   ß-Blockers, such as propranolol, bupranolol, metoprolol, nadoxolol, sotalol, alprenolol, oxprenolol, carteolol, labetalol, atenolol, pindolol, timolol and timolol maleate.

The preferred ß-blockers are timolol, bupranolol, timolol maleate and propranolol.

(b)   Antimicrobial agents, such as antibacterial, antifungal and antiviral agents are exemplified by lincomycin; clindamycin; tetracycline, oxytetracycline, chlorotetracycline and other tetracycline-type antibiotics; erythromycin;
2-thiopyridine N-oxide; halogen compounds, especially
iodine and iodine compounds; cephalosporins, i.e.,
any of the many new forms of these ß-lactam
antibiotics such as penicilline, penicillin G,
methacillin, carbenicillin and ticaricillin, and the
cephalosporins, cephalosporin C, cefazolin,
cephapirin, cephaloridine, cephalothin, cephapirin,
cephanone, cefamandole, cefaparole, cefoxitin,
cephacetrile, cefmetazole, cefoxitin, cefuroxime,
cefotaxime, T-1551, and the oxacephalosporin, S-6059;
any of the sulfonamide class of antibacterials;
streptomycin or any other members of the class.

The preferred agents are lincomycin,
tetracycline, erythromycin, penicillin, penicillin G,
cefoxitin, streptomycin, carbenicillin, cephapirin,
cephalosporin C and cephanone.

(c)   Steroidal anti-inflammatory agents,
such as corticosteroids, such as hydrocortisone,
hydrocortisone 17-valerate, hydrocortisone
17-butyrate, hydrocortisone 21-acetate, betamethasone
valerate, triamcinolone acetonide, fluocinonide,
desonide, fluocinolone acetonide, dexamethasone,
dexamethasone 21-phosphate, prednisolone,
prednisolone 21-phosphate, haloprednone, cortisone
acetate, hydrocortisone cyclopentylpropionate,
cortodoxone, flucetonide, fludrocortisone acetate,
flurandrenolone acetonide, medrysone, amcinafal,
amcinafide, betamethasome, betamethasone benzoate,
chloroprednisone acetate, clocortolone acetate,
descinolone acetonide, desoximetasone, dichlorisone
acetate, difluprednate, flucloronide, flumethasone, flumethasone pivalate, flunisolide acetate, fluocortolone, fluorometholone, fluperolone acetate, fluprednisolone, fluprednisolone valerate, meprednisone, methyl prednisolone, paramethasone acetate, prednisolamate, prednisone, prednival, triamcinolone, triamcinolone hexacetonide, cortivazol, formocortal and nivazol.

The preferred agents are hydrocortisone, hydrocortisone 21-acetate, betamethasone, betamethasone valerate, prednisolone, prednisolone 21-phosphate, cortisone acetate, fludrocortisone acetate, triamcinolone and nivazol.

(d)  Non-Steroidal anti-inflammatory agents, such as indomethacin, naproxen, fenoprofen, ibuprofen, alcolfenac, phenylbutazone, mefenamic acid, sulindac, desoxysulindac, diflunisal, aspirin, salicylamide, salicylic acid, flufenisal, salsalate, triethanolamine salicylate, aminopyrine, antipyrine, oxyphen-butazone, apazone, cintazone, flufenamic acid, clonixeril, clonixin, meclofenamic acid, flunixin, colchicine, demecolcine, allopurinol, oxypurinol, benzydamine hydrochloride, dimefadane, indoxole, intrazole, mimbane hydrochloride, paranylene hydrochloride, tetrydamine, benzindopyrine hydrochloride, fluprofen, ibufenac, ketoprofen, naproxol, fenbufen, cinchophen, diflumidone sodium, fenamole, flutiazin, metazamide, letimide hydrochloride, nexeridine hydrochloride, octazamide, molinazole neocinchophen, nimazole, proxazole citrate, tesicam, tesimide, tolmetin, tramadol and triflumidate.

The preferred agents are indomethacin, naproxen, fenaprofen, sulindac, ibuprofen, diflunisal, aminopyrine, antipyrine, nimazole, tramadol, fluprofen and demecolcine.

0147780

(e)   Antihypertensive agents such as clonidine and α-methyldopa, and antianginas such as propranolol hydrochloride, erythrityl tetranitrate, pentaerythritol tetranitrate, isosorbide dinitrate and dioxyline phasphate; vasodilator agents such as nitroglycerin, erythritol tetranitrate,  isosorbide dinitrate, mannitol hexanitrate, pentaerythrityl tetranitrate, papaverine and dipyridamole.

The preferred agents are methyldopa, clonidine and antianginas such as propranolol hydrochloride, erythrityl tetranitrate or dioxyline phosphate.

(f)   Sex hormones such as estrogens, androgens and progestins, especially the natural sex hormones estradiol, testosterone and progesterone.

The preferred agents are estrogen, progestin, androgen, testosterone and progesterone.

(g)   Muscle relaxants such as succinylcholine chloride, baclofen, dantrolene sodium, metaxalone, cyclobenzaprine hydrochloride and diazepam.

(h)   Antiasthma agents, such as theophylline, terbutaline sulfate, dyphyline, guaifenesin and cromoglycic acid and its prodrugs [described, for example, in International Journal of Pharmaceutics, 7, 63-75 (1980)].  Because cf its short half-life, cromoglycic acid is an especially desirable candidate for formulation with polyvinyl alcohol in accordance with the present invention.

(i)   Antimetic agents, such as pipamazine, chlorpromazine and dimenhydrinate.

(j) Antidepressant agents, such as protriptyline hydrochloride, amitriptyline, perphenazine and amitriptyline hydrochloride,

chlordiazepoxide hydrochloride, phenizine sulfate and doxepin hydrochloride.

The preferred agents are protriptyline hydrochloride, chlordiazepoxide hydrochloride, amitriptyline and doxepin hydrochloride.

(k)    Diuretics such as aldactone, diuril dyazide, enduron, hydrochlorothiazide and bretic.

The preferred agents are aldactone, diuril and hydrochlorothiazide.

(l)    Vasodilator agents such as nitroglycerin, erythritol tetranitrate, isosorbide dinitrate, mannitol hexanitrate, pentaerythrityl, papaverine and dipyridamole.

The preferred agents are nitroglycerin, erythritol tetranitrate, mannitol hexanitrate and papaverine.

Those skilled in the art will realize that the type of beneficial agent used in the core is not critical and that any beneficial agent can be used in accordance with the practice of this invention as long as is coated with polyvinyl alcohol.

The quantity of active agent necessary for preparing drug forms varies over a wide range but would normally be regulated by that quantity known in the art necessary to comprise a therapeutically effective unit dosage.  As stated previously, the thickness of the polyvinyl alcohol is inversely proportional to the desired rate of release of the drug.

The quantity of polyvinyl alcohol necessary for preparing drug forms may vary over a wide range. The quantity used in connection with the active agent in order to be effective in controlling the release of said active agent and rate limiting barrier for drug delivery systems is that quantity necessary to bring about the desired response. Generally, the amount of polyvinyl alcohol film coating for the entire drug delivery device (tablet, capsule, suppository & etc.) ranges from 1% to 15% by weight of the entire drug delivery device, preferably from 3% to 10%. The active or beneficial agent may range from 0.1 to 500 mg per dosage unit depending on the pharmaceutically recommended dosage of the specific active agent employed. Similiar dosage and composition apply where a combination (two or more) of active agents are employed.

Physiologically acceptable buffers or mixtures thereof such as primary, secondary or tertiary salts of phosphoric acid, salts of phthalic acid, citric acid, tartaric acid or amino acids or mixtures thereof are employed in the core portion of the drug delivery device.

Conventional lubricants such as magnesium stearate, stearic acid, magnesium lauryl sulfate can also be used in the core portion of the drug delivery device of the invention.

Other binders and fillers known in the art can be used in conjunction with the drug or beneficial agent in the preparation of the core.

The following examples illustrate preparation of various compositions of the invention. The examples should be construed as illustrations of the invention, rather than, limitations thereof.

EXAMPLE 1

Core Tablet

| Microcrystalline cellulose | 150 mg |
| L-dopa | 250 mg |
| Magnesium Stearate | 2 mg |

Blend to form a uniform distribution of microcrystalline cellulose and L-dopa. Sift the magnesium stearate onto the powder blend and mix for one to two minutes. Tablets are compressed using 3/8 biconvex round punches.

Coating

A.  Film Coating Solution

|  | Parts |
| Polyvinyl alcohol super-hydrolyzed | 2 |
| Water | 98 |

Disperse the polymer into the water, heat to boiling until complete solution occurs and cool to room temperature.

B.  Spray tablets to apply a coating of at least 1 to 5% of dried polymer weight on of the total tablet weight.

As an alternate to the tablet, the active agent can be prepared as granules or pellets and coated as described above.

## EXAMPLE 2

Core Tablet

| | |
|---|---|
| Microcrystalline cellulose | 150mg |
| Timolol Maleate | 10mg |
| Sodium Phosphate Dibasic | 2mg |
| Magnesium Stearate | 2mg |

Blend to form a uniform distribution of microcrystalline cellulose, sodium phosphate dibasic and timolol maleate.  Sift the magnesium stearate onto the powder blend and mix for one to two minutes.  Tablets are compressed using 3/8 biconvex round punches.

Coating

A.    Film Coating Solution            parts

Polyvinyl alcohol super-hydrolyzed ....2
Water                           98

Disperse the polymer into the water, heat to boiling until complete solution occurs and cool to room temperature.

B.    Spray tablets to apply a coating of at least 1 to 5% of coating solution of the total tablet weight.

As an alternate to the tablet, the active agent can be prepared as granules or pellets and coated as described above.

In like manner the following combinations of other active agents and polyvinyl alcohol can be formulated.

| Example | Active Agent | Polyvinyl Alcohol (%) by total weight |
|---------|--------------|---------------------------------------|
| 3 | Cefmetazole | 3 |
| 4 | Cefoxitin | 5 |
| 5 | Penicillin G | 5 |
| 6 | Carbenicillin | 2 |
| 7 | Methacillin | 8 |
| 8 | Bupranolol | 9 |
| 9 | Timolol | 10 |
| 10 | Timolol Maleate | 3 |
| 11 | Alprenolol | 4 |
| 12 | Lincomycin | 1 |
| 13 | Tetracycline | 5 |
| 14 | Erythromycin | 3 |
| 15 | Cefazolin | 10 |
| 16 | Hydrocortisone | 5 |
| 17 | Hydrocortisone 17-Valerate | 3 |
| 18 | Hydrocortisone 21-Acetate | 7 |
| 19 | Betamethasone Valerate | 5 |
| 20 | Dexamethasone | 4 |
| 21 | Prednisolone | 9 |
| 22 | Cortisone Acetate | 4 |
| 23 | Betamethasone | 3 |
| 24 | Indomethacin | 2 |
| 25 | Ibuprofen | 6 |
| 26 | Phenylbutazone | 4 |
| 27 | Sulindac | 2 |
| 28 | Aminopyrine | 8 |

| 29 | Demecolcine | 2 |
| 30 | Clonidine | 4 |
| 31 | L-Methyldopa | 2 |
| 32 | Erythritol Tetranitrate | 7 |
| 33 | Mannitol Hexanitrate | 9 |
| 34 | Estrogen | 2 |
| 35 | Androgen | 3 |
| 36 | Progestin | 4 |
| 37 | Estradiol | 6 |
| 38 | Testosterone | 5 |
| 39 | Cyclobenzaprine | 3 |
| 40 | Diazepam | 5 |
| 41 | Cromoglycic acid | 5 |
| 42 | Pipamazine | 7 |
| 43 | Chlorpromazine | 8 |
| 44 | Dimenhydrinate | 4 |
| 45 | Protriptyline hydrochloride | 10 |
| 46 | Chlordiazepoxide hydrochloride | 9 |
| 47 | Doxepin hydrocloride | 6 |
| 48 | Nitroglycerin | 8 |
| 49 | Erythritol tetranitrate | 4 |
| 50 | Isosorbide dinitrate | 3 |
| 51 | Papaverine | 2 |
| 52 | Hydrochlorothiazide | 3 |

## EXAMPLE 53

### Core Tablet

| Microcrystalline cellulose | 150 mg |
| L-dopa | 200 mg |
| carbidopa | 25 mg |
| Magnesium Stearate | 2 mg |

Blend to form a uniform distribution of microcrystalline cellulose, carbidopa and L-dopa. Sift the magnesium stearate onto the powder blend and mix for one to two minutes.  Tablets are compressed using 3/8 biconvex round punches.

Coating

A.    Film coating Solution

|                                          | Parts |
|------------------------------------------|-------|
| Polyvinyl alcohol super-hyrolyzed        | 12    |
| Water                                    | 88    |

Disperse the polymer into the water, heat to 95°C until complete solution occurs and cool to room temperature.

B.   Spray tablets to apply a coating of at least 1 to 5% of coating solution of the total tablet weight.

## EXAMPLE 54

The core tablets of Example 1 are coated with a solution containing 10 parts carbidopa, 12 parts polyvinyl alcohol and 78 parts water until a coating weight of 2.5 to 10% of tablet core weight.

As an alternate to the tablet, the active agent can be prepared as granules or pellets consisting of multiparticle dosage forms and coated as described above.

In like manner other combinations of other active agents and polyvinyl alcohol can be prepared.

## EXAMPLE 55

Suppository

A film coating solution of polyvinyl alcohol and water (88:12) on a suppository formed from standard excipients such as:

| | | |
|---|---|---|
| Indomethacin | 0.100 gm | |
| carbowax 1000 (polyethylene glycol 1000) | | |
| | 0.500 gm | |
| carbowax 4000 (Polyethylene glycol 4000) | | |
| | 1.800 gm | |

is applied using formulation and which consitutes from 0.2 to 2% of formulation weight coating solution, as described for tablets.

In like manner other combinations of active agents and polyvinyl alcohol in suppository form can be prepared.

| EXAMPLE | Active Agent | Polyvinyl Alcohol (%) |
|---|---|---|
| 56 | Cefmetazole | 10 |
| 57 | Cefoxitin | 8 |
| 58 | Timolol | 12 |
| 59 | Timolol Maleate | 14 |
| 60 | Hydrocortisone | 5 |
| 61 | Hydrocortisone 21-Acetate | 7 |
| 62 | Indomethacin | 2 |
| 63 | Sulindac | 6 |
| 64 | Cyclobenzaprine | 7 |

WHAT IS CLAIMED IS:

1. A composition of matter for oral, rectal or vaginal administration comprising a core tablet or granules of a therapeutically effective amount of at least one therapeutic agent selected from the group consisting of a ß-blocker, antimicrobial, steroidal anti-inflammatory, non-steroidal anti-inflammatory, antihypertensive, sex hormone, muscle relaxant, antiasthma, antiemetic, antidepressant, diuretic and vasodilator, and a polyvinyl alcohol coating on said core tablet or granules, the amount of said therapeutic agent and polyvinyl alcohol constituting from 0.1 to 500 mg per composition and 1 to 15% of total composition weight, respectively.

2. The composition of Claim 1, wherein said ß-blocker is selected from the group consisting of timolol, bupranolol, timolol aleate, or propranolol; said antimicrobial is selected from the group consisting of lincomycin, tetracycline, erythromycin, penicillin, penicillin G, cefoxitin, streptomycin, carbenicillin, cephapirin, cephalosporin C or cephanone; said steroidal anti-inflammatory is selected from the group consisting of hydrocortisone, hydrocortisone 21-acetate, betamethasone, betamethasone valerate, prednisolone, prednisolone 21-phosphate, cortisone acetate, fludrocortisone acetate, triamcinolone or nivazol; said non-steroidal anti-inflammatory is selected from the group consisting of indomethacin, naproxen, fenoprofen, sulindac, ibuprofen, diflunisal, aminopyrine, antipyrine, nimazole, tramadol, fluprofen or demecolcine; said antihypertensive is selected from the group consisting of methyldopa, clonidine and antianginas such as propranolol hydrochloride, erythrityl tetranitrate or dioxyline phosphate; said sex hormone is selected from the group consisting of estrogen, progestin, androgen, testosterone or progesterone; said muscle relaxant is selected from the group consisting of succinylcholine chloride, baclofen, dantrolene sodium, metaxalone, cyclobenzoprine hydrochloride or diazepam; said anti-asthma is selected from the group consisting of theophylline, terbutaline sulfate, dyphyline and guaifenesin, or cromoglycic acid; said antiemetic is selected from the group consisting of chlorpromazine, dimenhydrinate or pipamazine; said antidepressant is selected from the group consisting of protriptyline hydrochloride, chlordiazepoxide hydrochloride, amitriptyline, or doxepin hydrochloride; said diuretic is selected from the group consisting of aldactone, diuril or hydrochlorothiazide; and said vasodilators such as nitroglycerin, erythritol tetranitrate, isosorbide dinitrate, mannitol hexanitrate, pentaerythrityl tetranitrate, papaverine and dipyridamole, or mixture thereof and wherein said polyvinyl alcohol constitutes from 2 to 5% of the composition weight.

3.    The composition of Claim 2 wherein said ß-blocker is timolol maleate, said antimicrobial is cefoxitin, said steroidal anti-inflammatory is hydrocortisone, said non-steroidal anti-inflammatory is indomethacin, said antihypertensive is methyldopa, said sex hormone is estrogen and said anti-depressant is amitriptyline.

4. The composition of Claim 1, wherein said therapeutic agent is a mixture of timolol maleate and hydrochlorothiazide.

5. The composition of Claim 1, wherein said therapeutic agent is a mixture of hydrocortisone and indomethacin.

6. The composition of Claim 1, wherein said therapeutic agent is a mixture of indomethacin and cyclobenzaprine.

7. The composition of Claim 1, wherein said therapeutic agent is a mixture of methyldopa and hydrochlorothiazide or a mixture of amitriptyline and bupranolol.

8. The composition of Claim 1 in oral or suppository form.

9. The composition of Claim 1 further comprising at least one pharmaceutically acceptable buffer.